**Europäisches Patentamt**

(19) **European Patent Office**

**Office européen des brevets**

(11) Veröffentlichungsnummer: **0 249 870 B1**

(12) **EUROPÄISCHE PATENTSCHRIFT**

(45) Veröffentlichungstag der Patentschrift:
13.03.91 Patentblatt 91/11

(51) Int. Cl.$^5$: **C07C 19/02**, C07C 17/38,
C07C 17/16

(21) Anmeldenummer: **87108376.2**

(22) Anmeldetag: **10.06.87**

(54) **Verfahren zur Beseitigung von Dimethylether in Methylchlorid.**

(30) Priorität: **14.06.86 DE 3620069**

(43) Veröffentlichungstag der Anmeldung:
**23.12.87 Patentblatt 87/52**

(45) Bekanntmachung des Hinweises auf die
Patenterteilung:
**13.03.91 Patentblatt 91/11**

(84) Benannte Vertragsstaaten:
**BE DE ES FR GB IT NL**

(56) Entgegenhaltungen:
**CH-A- 495 932**
**DE-A- 2 658 132**
**US-A- 1 920 846**

(73) Patentinhaber: **HOECHST**
**AKTIENGESELLSCHAFT**
**Postfach 80 03 20**
**W-6230 Frankfurt am Main 80 (DE)**

(72) Erfinder: **Post, Hendrik Willem**
**Paulinenweg 6**
**W-6238 Hofheim am Taunus (DE)**
Erfinder: **von Piessen, Helmold, Dr.**
**Kugelhermstrasse 16**
**W-6240 Königstein/Taunus (DE)**
Erfinder: **Lendle, Wilhelm, Dr.**
**Hirschpfad 5**
**W-6232 Bad Soden am Taunus (DE)**

## Beschreibung

Die vorliegende Erfindung betrifft ein Verfahren zur Beseitigung von Dimethylether aus rohem Methylchlorid, das bei der katalytischen Methanol-Veresterung anfällt, durch katalytische Spaltung mittels Chlorwasserstoff.

Ein wichtiges Verfahren zur Herstellung von Methylchlorid beruht auf der Veresterung von Methanol mit Chlorwasserstoff bei erhöhter Temperatur an einem Feststoffkatalysator. Im allgemeinen arbeitet man bei 350 bis 400°C und verwendet einen Aluminiumoxid-Katalysator. Die dabei ablaufende Reaktion wird durch die folgende Gleichung wiedergegeben.

$$CH_3OH + HCl \rightarrow CH_3Cl + H_2O$$

Als Nebenprodukt entstehen wechselnde Mengen Dimethylether (DME), die mit dem gasförmigen Methylchlorid aus der Reaktionszone abgeführt werden. Nach Abkühlung des Gasgemisches auf Raumtemperatur und Kondensation von Wasser enthält das verbleibende Methylchlorid noch Methanol (unter 0,03%), 0,3 bis 2% DME, unter 2% Chlorwasserstoff und ca. 0,1% Wasser. Üblicherweise wird dieses rohe Methylchlorid mit Schwefelsäure von mindestens 80 Gew.-% $H_2SO_4$ gewaschen und so getrocknet. Dabei wird nicht nur das restliche Wasser von der Schwefelsäure gebunden, sondern auch Spuren von Methanol und fast der gesamte Dimethylether. Meist wird zur Trocknung 95%ige Schwefelsäure eingesetzt. Nach Erschöpfung muß die Waschsäure, die dann noch ca. 80%ig ist und 5 bis 10% DME (gebunden) enthält, aufgearbeitet werden.

Die Aufarbeitung dieser Abfallschwefelsäure ist ein schwierig zu lösendes Umweltproblem und es sind mehrere Verfahren zu seiner Lösung vorgeschlagen worden. So kann man z.B. gemäß DE-OS 31 51 691 die Abfallschwefelsäure bei Siedetemperatur mit Dampf ausblasen und die kondensierten Dämpfe zur Entfernung der organischen Bestandteile einer biologischen Abwasserreinigungsanlage zuführen. Die rückgewonnene Schwefelsäure kann konzentriert und, falls nötig, weiter gereinigt werden.

DME muß als Verunreinigung aus dem Methylchlorid entfernt werden. Wegen des nahezu gleichen Siedepunkts ist eine destillative Abtrennung schlecht möglich. Es bestand daher die Aufgabe, ein verbessertes Verfahren zur Entfernung von DME aus rohem Methylchlorid aufzufinden.

Es wurde nun ein Verfahren zur Beseitigung von Dimethylether, der als Verunreinigung in rohem Methylchlorid enthalten ist, das bei der katalytischen Methanolveresterung anfällt, gefunden, das dadurch gekennzeichnet ist, daß man das rohe Methylchlorid abkühlt, so daß sich Wasser ganz und Chlorwasserstoff mindestens teilweise abscheiden, man anschließend das Methylchlorid im Gemisch mit gasförmigen Chlorwasserstoff bei Temperaturen von 130 bis 450°C über einen Methanol-Veresterungskontakt leitet, wobei das Molverhältnis Chlorwasserstoff/DME mindestens 2 : 1 beträgt. Je höher dieses Molverhältnis umso niedrigere Restgehalte an Dimethylether lassen sich erreichen. Bevorzugt sind Molverhältnisse von 2 : 1 bis 10 : 1.

Durch das erfindungsgemäße Verfahren läßt sich die Menge an verbleibendem Dimethylether im Methylchlorid wesentlich verringern. Sofern eine weitere Reinigung mit Schwefelsäure durchgeführt wird, ergibt sich eine deutliche Verringerung der Menge an benötigter Schwefelsäure und anfallendem Kondensat mit organischen Verunreinigungen, die entsorgt werden müssen.

Eine Beschreibung der Methanolveresterung findet sich in Winnacker-Küchler, Chemische Technologie, 4. Aufl., Bd. 6 (1982), S. 4.

Aus der US-PS 2 080 710 ist es bekannt, Dialkylether mit wäßriger Salzsäure und Zinkchlorid unter Bildung von Alkylchloriden zu spalten. Dieses Verfahren wird jedoch in wäßrig-flüssiger Phase in Abwesenheit von überschüssigem Alkylchlorid und bei relativ niedrigen Temperaturen durchgeführt. Demgegenüber erfolgt nach dem erfindungsgemäßen Verfahren die Spaltung des Dimethylether enthaltenden Gemisches bei 130 bis 450°C, vorzugsweise 150 bis 400°C in Gegenwart großer Mengen Methylchlorid. Vorzugsweise liegt die Untergrenze des verwendeten Temperaturbereichs bei 200°C, insbesondere 250°C. Eine bevorzugte Obergrenze liegt unterhalb 350°C, insbesondere unterhalb 300°C. Je niedriger die Temperatur, umso geringer die Rußbildung und umso aktiver sollte der Katalysator, bzw. umso länger sollten die Verweilzeiten sein. Die hierbei eingesetzten Methanolveresterungs-Kontakte sind auch in der Lage, Dimethylether in Gegenwart von Chlorwasserstoff zu spalten. Verwendbar sind beispielsweise Eisen(III)-chlorid, Zinkchlorid, Cadmiumchlorid, Aluminiumoxid und Zeolithe. Bevorzugt sind schwermetallfreie Kontakte, insbesondere Aluminiumoxid. Es ist überraschend, daß nach dem erfindungsgemäßen Verfahren der niedrige DME-Gehalt des rohen Methylchlorids durch nochmalige Einwirkung des Katalysators erheblich vermindert werden kann und sogar DME-Gehalte im ppm-Bereich erhalten werden können.

Nach der erfindungsgemäßen Spaltung von Dimethylether kann man das behandelte Gas erneut abkühlen, um Wasser und Chlorwasserstoff abzuscheiden. Anschließend kann man das von Dimethylether befreite Gas mit Schwefelsäure einer Konzentration von ca. 80 bis 96 Gew.-% $H_2SO_4$ waschen und die letzten Spuren an DME und Wasser entfernen. Auch hierbei soll in der Waschsäure ein Gehalt an (gebundenem) DME von 5 bis 10% nicht überschritten werden.

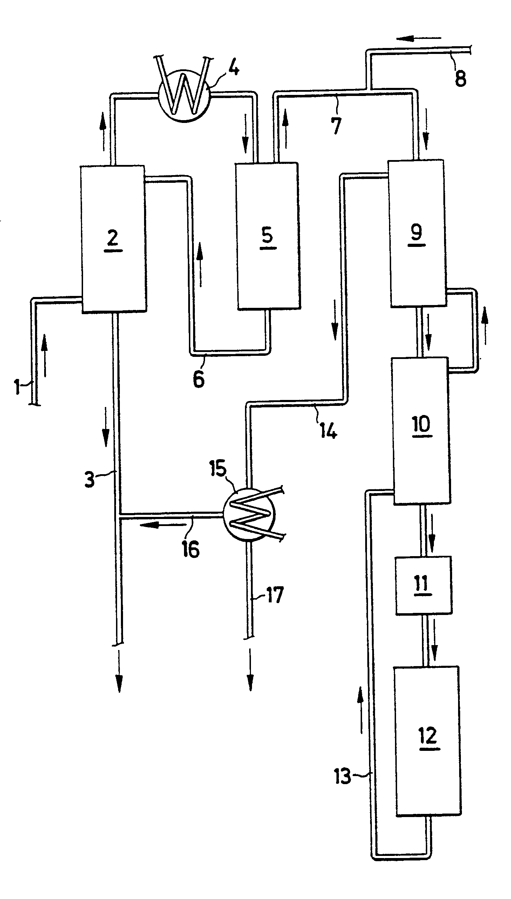

Die Durchführung des Verfahrens wird anhand der Figur näher erläutert. Das aus der Methanolveresterung mit ca. 300°C kommende Gasgemisch wird durch Leitung (1) in die Füllkörperkolonne (2) eingespeist und dort mit 35-40%iger Salzsäure berieselt. Das Gas wird dabei auf 125°C abgekühlt. Gleichzeitig bildet sich eine 20-25%ige Salzsäure (Quenchsäure), die durch Leitung (3) abgezogen wird. Das Gasgemisch wird anschließend in einem Kühler (4) aus Graphit weiter auf 40-45°C abgekühlt. Kondensiertes Wasser und Chlorwasserstoff werden als 35-40%ige Salzsäure abgetrennt und sammeln sich im Behälter (5) an. Durch Leitung (6) wird diese Salzsäure in die Füllkörperkolonne (2) zurückgeführt. Das Rohgas verläßt den Behälter (5) durch Leitung (7). Es enthält neben Dimethylether noch 0,1 bis 0,2% Wasser und 0,3 bis 4% Chlorwasserstoff. Durch Leitung (8) kann Chlorwasserstoff zugegeben werden, um das Molverhältnis Chlorwasserstoff/Dimethylether zu erhöhen. Bei einem Dimethylethergehalt von durchschnittlich 0,5 Vol.-% wird ein Gehalt von mindestens 1 Vol.-% HCl eingestellt. Das Gas gelangt in einen Wärmetauscher (9) aus Graphit, dann in einen weiteren Wärmetauscher (10) aus Nickel und schließlich in ein aus Nickel bestehendes Heizaggregat (11), worin es auf eine Reaktionstemperatur zwischen 150 und 400°C erwärmt wird. Anschließend wird es im Reaktor (12) über den Katalysator geführt. Das gebildete Reaktionsgemisch wird durch Leitung (13) in die Wärmetauscher (10) und dann (9) geführt. Dort überträgt es seinen Wärmeinhalt auf das zuströmende Gas. Das Reaktionsgemisch verläßt (9) durch Leitung (14). Nach restlicher Kühlung mit Wasser in einem Wärmetauscher (15) aus Graphit werden erneut Wasser und Chlorwasserstoff als Salzsäure durch Leitung (16) abgetrennt. Die Salzsäure fließt durch Leitung (16) der Leitung (3) zu. Das behandelte Gas wird durch Leitung (17) der Schwefelsäurewäsche (nicht gezeichnet) zugeführt.

Man kann auch die Methanol-Veresterung mit erhöhtem Gehalt an HCl betreiben, so daß nach Kondensation von Wasser noch freier HCl (z.B. 1 bis 6 Vol.-%) vorhanden ist. In diesem Fall ist eine nachträgliche Zugabe von HCl nich mehr erforderlich.

Bei der Ausübung des Verfahrens in technischen Anlagen tritt ab 400°C bei längeren Reaktionszeiten teilweise Zersetzung unter Rußabscheidung ein. Hierbei bewährte sich ein Arbeiten mit einem $Al_2O_3$-Katalysator (Harshaw) bei 130 bis 300°C, vorzugsweise 200 bis 250°C.

Die Erfindung wird ferner durch das folgende Beispiel erläutert.

## Beispiel

Es wurde eine Apparatur aufgebaut, die aus einem Vorwärmer, einem elektrisch beheizten Reaktor, mehreren Kühlern und einer Gaswäscheapparatur bestand. Die Menge des austretenden Gases wurde durch eine Gasuhr gemessen.

Als Vorwärmer wurde ein Glaskolben verwendet, der sich in einem Heizbad befand. Der sich anschließende elektrisch beheizte Reaktor bestand aus einem mit Katalysator gefüllten Nickelrohr (Länge 700 mm, Durchmesser 50 mm). Als Katalysator wurde Aluminiumoxid der Fa. Alcoa (Katalysator F-1/1/4-6) verwendet, das üblicherweise für die Methanolveresterung eingesetzt wird.

Die Versuchstemperaturen betrugen 340 bis 360°C. Es folgte ein Luftkühler aus Glas, dann ein Kühler, der mit einer Flüssigkeit von 0°C betrieben wurde und ebenfalls aus Glas bestand. Als Gaswäsche zur Beseitigung von Chlorwasserstoff diente eine kleine Füllkörperkolonne, die mit Wasser (200 ml/h) beschickt wurde. Die Zusammensetzung der eintretenden und austretenden Gase wurde gaschromatographisch bestimmt. Die Mengen an Methylchlorid, Chlorwasserstoff und Dimethylether finden sich in der folgenden Tabelle.

| Einsätze | $CH_3Cl$ mol/h | HCl im rohen $CH_3Cl$ enthalten, mol/h | HCl zugesetzt mol/h | DME mol/h |
|---|---|---|---|---|
| Versuch 1 | 5,30 | 0,022 | - | 0,061 |
| Versuch 2 | 5,35 | 0,083 | 0,087 | 0,036 |
| Versuch 3 | 5,39 | 0,086 | 0,462 | 0,037 |
| Versuch 4 | 5,36 | 0,122 | 1,203 | 0,030 |

| Ausbeuten | $CH_3Cl$ mol/h | HCl mol/h | DME mol/h | Versuchs-temp. | Versuchs-temp. |
|---|---|---|---|---|---|
| Versuch 1 | 5,35 | ca. 0,0 | 0,0073 | 352°C | 1 h |
| Versuch 2 | 5,38 | ca. 0,1 | 0,0033 | 342°C | 2 h |
| Versuch 3 | 5,42 | ca. 0,5 | 0,0011 | 359°C | 1 h |
| Versuch 4 | 5,39 | ca. 1,3 | 0,0003 | 358°C | 3 h |

| | $\dfrac{\text{mol HCl}}{\text{mol } CH_3Cl}$ im Einsatzgemisch | $\dfrac{\text{mol DME (Endprodukt)}}{\text{mol DME (Einsatzgemisch)}}$ |
|---|---|---|
| Versuch 1 | 0,004 | 0,12 |
| Versuch 2 | 0,032 | 0,09 |
| Versuch 3 | 0,102 | 0,03 |
| Versuch 4 | 0,247 | 0,01 |

Es zeigt sich, daß der Anteil des im rohen Methylchlorid enthaltenen Dimethylethers mit Hilfe des erfindungsgemäßen Verfahrens um 88 bis 99% vermindert wurde.

**Ansprüche**

1. Verfahren zur Beseitigung von Dimethylether der als Verunreinigung in rohem Methylchlorid enthalten ist, das bei der katalytischen Methanol-Veresterung anfällt, dadurch gekennzeichnet, daß man das rohe Methylchlorid abkühlt, so daß sich Wasser ganz und Chlorwasserstoff mindestens teilweise abscheiden, man anschließend das Methylchlorid im Gemisch mit gasförmigem Chlorwasserstoff bei Temperaturen von 130 bis 450°C über einen Methanol-Veresterungskontakt leitet, wobei das Molverhältnis Chlorwasserstoff/Dimethylether mindestens 2 : 1 beträgt.

2. Verfahren nach Anspruch 1, dadurch gekennzeichnet, daß man das über den Methanol-Veresterungskontakt geleitete Gas erneut abkühlt, um Wasser und Chlorwasserstoff abzuscheiden.

3. Verfahren nach Anspruch 2, dadurch gekennzeichnet, daß man das von Wasser und Chlorwasserstoff befreite Gas anschließend mit Schwefelsäure einer Konzentration von ca. 80 bis 96 Gew.-% $H_2SO_4$ wäscht, um die letzten Spuren an Dimethylether zu entfernen.

4. Verfahren nach Anspruch 1, dadurch gekennzeichnet, daß Aluminiumoxid als Veresterungskatalysator eingesetzt wird.

5. Verfahren nach Anspruch 4, dadurch gekennzeichnet, daß man bei Reaktionstemperaturen von 150 bis 400°C arbeitet.

**Claims**

1. A process for the removal of dimethyl ether which is present as an impurity in crude methyl chloride produced by the catalytic esterification of methanol, which comprises cooling the crude methyl chloride so that all the water and at least some of the hydrogen chloride separate out, and subsequently passing the methyl

4

chloride mixed with gaseous hydrogen chloride over a methanol esterification contact catalyst at temperatures from 130 to 450°C, the molar ratio of hydrogen chloride to dimethyl ether being at least 2 : 1.

2. The process as claimed in claim 1, wherein the gas passed over the methanol esterification contact catalyst is cooled again in order to deposit water and hydrogen chloride.

3. The process as claimed in claim 2, wherein the gas freed from water and hydrogen choloride is then washed with sulfuric acid having a concentration of approx. 80 to 96% by weight of $H_2SO_4$, in order to remove the last traces of dimethyl ether.

4. The process as claimed in claim 1, wherein aluminium oxide is employed as the esterification catalyst.

5. The process as claimed in claim 4, wherein the reaction is carried out at temperatures from 150 to 400°C.

## Revendications

1. Procédé pour éliminer l'oxyde de diméthyle contenu, en tant qu'impureté, dans le chlorure de méthyle brut obtenu lors de l'estérification catalytique du méthanol, procédé caractérisé en ce qu'on refroidit le chlorure de méthyle brut de telle façon que l'eau se sépare totalement et le chlorure d'hydrogène au moins partiellement, puis on fait passer le chlorure de méthyle, en mélange avec du chlorure d'hydrogène gazeux, à des températures de 130 à 450°C, sur un catalyseur d'estérification du méthanol, le rapport molaire du chlorure d'hydrogène au DME étant d'au moins 2 : 1.

2. Procédé selon la revendication 1 caractérisé en ce qu'on refroidit à nouveau le gaz que l'on a fait passer sur le catalyseur d'estérification du méthanol, cela pour séparer l'eau et le chlorure d'hydrogène.

3. Procédé selon la revendication 2 caractérisé en ce qu'on lave ensuite au moyen d'un acide sulfurique d'une concentration comprise entre environ 80 et 96% en poids d'$H_2SO_4$, le gaz débarrassé de l'eau et du chlorure d'hydrogène, cela pour éliminer les dernières traces d'oxyde de diméthyle.

4. Procédé selon la revendication 1 caractérisé en ce qu'on utilise de l'alumine comme catalyseur d'estérification.

5. Procédé selon la revendication 4 caractérisé en ce qu'on opère à des températures réactionnelles comprises entre 150 et 400°C.